# EUROPEAN PATENT SPECIFICATION

(11) **EP 2 566 483 B1**
(45) Date of publication and mention of the grant of the patent: **11.09.2019**
(21) Application number: 11778468.6
(22) Date of filing: 06.05.2011
(51) Int. Cl.: A61K 31/7008, A61P 17/00, A61K 31/7036, A61K 38/39

(54) **METHODS AND AGENTS FOR ENHANCING WOUND HEALING**
VERFAHREN UND MITTEL FÜR VERBESSERTE WUNDHEILUNG
PROCÉDÉS ET AGENTS POUR FAVORISER LA CICATRISATION

(30) Priority: 06.05.2010 US 332108 P
(43) Date of publication of application: 13.03.2013
(73) Proprietor: University Of Southern California, USC Stevens, Los Angeles, CA 90015 (US)
(72) Inventor: CHEN, Mei, Altadena CA 91001 (US); WOODLEY, David, Altadena CA 91001 (US)
(74) Representative: Viering, Jentschura & Partner mbB Patent- und Rechtsanwälte
(86) International application number: PCT/US2011/035653
(87) International publication number: WO 2011/140526

(56) References cited:
- WO-A1-2008/082821
- US-A1- 2001 051 607
- US-A1- 2005 261 210
- JEMIMA E. MELLERIO ET AL: "Medical management of epidermolysis bullosa: Proceedings of the IInd International Symposium on Epidermolysis Bullosa, Santiago, Chile, 2005", INTERNATIONAL JOURNAL OF DERMATOLOGY, vol. 46, no. 8, 1 August 2007 (2007-08-01) , pages 795-800, XP055080984, ISSN: 0011-9059, DOI: 10.1111/j.1365-4632.2007.03316.x
- RIGONI GURTLER T G ET AL: "Recessive dystrophic epidermolysis bullosa mitis - Case report", ANAIS BRASILEIROS DE DERMATOLOGIA, ECN-ED. CIENTIFICA NACIONAL, RIO DE JANEIRO, BR, vol. 80, no. 5, 1 September 2005 (2005-09-01), pages 503-508, XP009172938, ISSN: 0365-0596
- WOODLEY D T ET AL: "Injection of recombinant human type VII collagen restores collagen function in dystrophic epidermolysis bullosa", NATURE MEDICINE, NATURE PUBLISHING GROUP, NEW YORK, NY, US, vol. 10, no. 7, 1 July 2004 (2004-07-01), pages 693-695, XP008108910, ISSN: 1078-8956, DOI: 10.1038/NM1063 [retrieved on 2004-06-13]
- MAKI GOTO ET AL: "Targeted Skipping of a Single Exon Harboring a Premature Termination Codon Mutation: Implications and Potential for Gene Correction Therapy for Selective Dystrophic Epidermolysis Bullosa Patients", JOURNAL OF INVESTIGATIVE DERMATOLOGY, vol. 126, no. 12, 15 June 2006 (2006-06-15), pages 2614-2620, XP055019888, ISSN: 0022-202X, DOI: 10.1038/sj.jid.5700435
- WEINSTEIN J ET AL: "Aminoglycosides restore type VII collagen function by overcoming premature stop mutations: implications for DEB therapy", JOURNAL OF INVESTIGATIVE DERMATOLOGY, vol. 130, no. Suppl. 1, April 2010 (2010-04), page S92, XP002713667, & ANNUAL MEETING OF THE SOCIETY-FOR-INVESTIGATIVE-DERMATOLOGY; ATLANTA, GA, USA; MAY 05 -08, 2010 ISSN: 0022-202X
- WEINSTEIN ET AL.: 'Aminoglycosides Restore Type VII Collagen Functon by Overcoming Premature Stop Mutations: Implications for Dystrophic Epidermolysis Bullosa Therapy.' ABSTRACT BOOK: MEDICAL STUDENT RESEARCH FORUM AND POSTER DAY., [Online] 05 April 2010, page 2 Retrieved from the Internet: <URL:http://medweb.usc.edu/research/Researc h-Day-Program-2010.pdf> [retrieved on 2011-07-06]
- WOODLEY ET AL.: 'Intravenously Injected Human Fibroblasts Home to Skin Wounds, Deliver Type VII Collagen, and Promote Wound Healing.' MOLECULAR THERAPY. vol. 15, no. 3, 2007, pages 628 - 635, XP008159718
- JON COGAN ET AL: "Aminoglycosides Restore Full-length Type VII Collagen by Overcoming Premature Termination Codons: Therapeutic Implications for Dystrophic Epidermolysis Bullosa", MOLECULAR THERAPY, vol. 22, no. 10, 23 October 2014 (2014-10-23), pages 1741-1752, XP055321827, GB ISSN: 1525-0016, DOI: 10.1038/mt.2014.140

## Description

### CROSS-REFERENCE TO RELATED APPLICATIONS

This application claims the benefit of U.S. Provisional Application Number 61/332,108, file on May 6, 2010. The above applications are hereby incorporated herein by reference.

### STATEMENT REGARDING FEDERALLY SPONSORED RESEARCH AND DEVELOPMENT

This invention was made with government support under Contract Nos. RO1 AR47981 and RO1 AR33625 awarded by the National Institute of Health. The government has certain rights in the invention.

### FIELD OF THE INVENTION

The invention relates in general to treatment of subjects suffering from nonsense mutation in the *COL7A1* gene. More specifically, the invention provides compositions and methods for treatment of dystrophic epidermolysis bullosa due to nonsense mutation in the *COL7A1* gene.

### BACKGROUND OF THE INVENTION

Epidermolysis bullosa (EB) is a group of genetic conditions that cause the skin to be very fragile and to blister easily. Patients who suffer from EB can easily form blisters and skin erosions in response to minor injury or friction, such as rubbing or scratching. Dystrophic epidermolysis bullosa (DEB) is one of the major forms of EB. The signs and symptoms of this condition vary widely among affected individuals. In mild cases, blistering may primarily affect the hands, feet, knees, and elbows. In severe cases, widespread blistering may lead to vision loss, disfigurement, and other serious medical problems.

Researchers classify DEB into three major types: autosomal recessive dystrophic epidermolysis bullosa, Hallopeau-Siemens type (RDEB-HS), non-Hallopeau-Siemens type autosomal recessive epidermolysis bullosa (non-HS RDEB), and autosomal dominant type epidermolysis bullosa (DDEB). Although the three types vary in degree of severity, they have substantially overlapping clinical features and are caused by mutations in the same gene.

Of the three types, RDEB-HS is the most severe, classic form of the condition. Infants who are affected by this condition often are born with widespread blistering and areas of missing skin as a result of trauma during birth. Most often, blisters are presented over the whole body and may affect mucous membranes such as the moist lining of the mouth and digestive tract. As the blisters heal, they result in sever scaring. Scaring in the mouth and esophagus can make it difficult for the infant to chew and swallow food, leading to chronic malnutrition and slow growth. Additional complications of progressive scaring can include fusion of the fingers and toes, loss of fingernails and toenails, joint deformities (contractures) that restrict movement, and eye inflammation leading to vision loss. Additionally, young adults with this classic form of DEB have a very high risk of developing a form of skin cancer called squamous cell carcinoma, which tends to be unusually aggressive and is often life-threatening.

Non-HS RDEB is somewhat less severe than HS RDEB. It may be further divided into a range of subtypes. Blistering is limited to the hands, feet, knees, and elbows in mild cases, but may be widespread in more severe cases. Affected people often have malformed fingernails and toenails. Non-HS RDEB involves scarring in the areas where blisters occur, but this form of the condition does not cause the severe scarring characteristic of the HS RDEB.

DDEB tends to be milder than the RDEBs, with blistering often limited to the hands, feet, knees, and elbows. The blistering heal with scarring but is less severe. Most people afflicted with this condition have malformed fingernails and toenails. Their nails may be lost over time. However, in the mildest cases, abnormal nails are the only sign of the condition.

Considered together, the incidence of all types of DEB is estimated to be 6.5 per million newborns in the United States. The severe autosomal recessive forms of this disorder affect fewer than 1 per million newborns.

As mentioned above, all EBs are caused by genetic mutations in a gene. For all three types of DEBs, the mutations is in the *COL7A1* gene. This gene provides instructions for making a protein that is used to assemble type VII collagen (C7). Collagens are molecules that give structure and strength to connective tissues, such as skin, tendons, and ligaments throughout the body. Type VII collagen plays an important role in strengthening and stabilizing the skin. It is the main component of structures called anchoring fibrils, which anchor the top layer of skin, called the epidermis, to an underlying layer called the dermis.

*COL7A1* mutations alter the structure or disrupt the production of C7, which impairs its ability to help connect the epidermis to the dermis. In particular, about 25% of DEB cases are caused by nonsense mutations leading to premature termination condons (PTCs), which give rise to either no C7 or a truncated C7. When C7 is abnormal or missing, friction or other minor trauma can cause the two skin layers to separate. This separation leads to the formation of blisters, which can cause extensive scarring as they heal.

At the molecular level, C7 is composed of three identical alpha chains, each consisting of a 145-kDa central collagenous triple-helical segment, flanked by a large 145-kDa amino-terminal, non-collagenous domain (NC1), and a small 34-kDa carboxyl-terminal non-collagenous domain (NC2) (Burgeson, J Invest Dermatol 101: 252-255 (1993); Sakai et al., J Cell Biol 103: 1577-1586 (1986), the contents of both references are incorporated herein by reference). Within the extracellular space, C7 molecules form antiparallel dimers which aggregate laterally to form anchoring fibrils. In normal skin, C7 forms anchoring fibrils ranging from about 200 - 700 nm in size that emanate from epidermal-dermal junction (EDJ) and extend perpendicularly down into the papillary dermis. In DEB patients, the EDJ is characterized by a paucity of normal anchoring fibrils. Based on the underlying etiology of the disease, one logical approach for treating the disease is to correct the genetic defect through gene therapy.

Several attempts to treat DEB has been reported recently. In the study by Oritz-Urda et al. (Nat Med 8: 1166-1170 (2002), the content of which is incorporated herein by reference), *COL7A1* cDNA was successfully and stably integrated into C7-null keratinocytes from recessive DEB (RDEB) patients *ex vivo* using a phi C31 integrase-based non-viral gene transfer approach. By transplanting a human skin equivalent comprising these gene-corrected cells onto severe combined immunodeficient (SCID) mice, they showed that many of the RDEB features were corrected after gene transfer.

In another attempt, a minimal lentiviral vector was developed to express C7 in RDEB keratinocytes and fibroblasts (in which C7 was absent). This construct was subsequently used to demonstrate the reversion of the RDEB cellular phenotype (Chen et al., Nat Genet 32: 670-675 (2002), the content of which is incorporated herein by reference). In this experiment, the gene-corrected RDEB cells and native un-corrected RDEB cells were used to create a composite human skin equivalent which was then transplanted onto SCID mice. It was shown that the transplanted human skin made with the gene-corrected RDEB cells (but not the control un-corrected RDEB cells) exhibited C7 at the EDJ and the RDEB skin phenotype was corrected. Moreover, in the skin equivalents composed of gene-corrected (but not gene-uncorrected) cells, the transgene-derived C7 also created anchoring fibril structures that were correctly organized into the basement membrane zone (BMZ) lying between the epidermis and dermis.

However, these types of *ex vivo* approach requires transplantation of gene-corrected cells onto surgically prepared sites of the patient's skin. The experience of using cultured keratinocyte autografts for transplantation onto human wounds had shown that they are often fraught with technical difficulties and poor graft take. Therefore, although these type of *ex vivo* gene therapy (i.e. gene correcting cells in culture and then transplanting them back as skin equivalents onto the DEB patient) are theoretically possible, the technical hurdles make them in-efficient, logistically difficult, expensive, labor-intensive and only of limited efficacy.

As an alternative approach, a more straightforward "direct *in vivo* gene therapy" has also been reported. With this approach, DEB wounded skin is directly injected through intradermal injection with gene-corrected RDEB fibroblasts. The gene-corrected intradermally injected cells then set up residence in the DEB skin and synthesize and secrete C7 which is lacking in the DEB skin. Surprisingly, the secreted C7 in the extracellular dermal tissue, binds to the BMZ of the DEB skin and correctly organizes into anchoring fibril structures. Now, the DEB skin which previously lacked C7 and anchoring fibrils, now has these elements and the DEB skin phenotype is corrected. The poor epidermal-dermal adherence is now corrected. This is called "cell therapy" for DEB.

It yet another approach, it has also been showed that the same events would occur if full-length or "mini-C7" intradermally injected into DEB skin. In such cases, the injected C7 would bind to the BMZ of the DEB skin and form correctly-organized anchoring fibrils and correct the DEB skin phenotype. The is called "protein therapy" for DEB.

Because patients with severe DEB have widespread lesions and multiple wounds spanning large areas of trauma-prone sites such as the sacrum, hips, feet, mouth, scalp, lower back and hands, the treatment of such DEB patients via any of the three above outlined direct intradermal injection approaches would require numerous injections into multiple wound sites. Accordingly, intradermal injections of the therapeutic agents outlined above (gene-corrected cells, recombinant forms of C7 or C7 expressing vectors) would require site-specific treatment of each and every wound by one or more intradermal injections. While this is doable, such a cumbersome method of treatment still leaves much to be desired.

Despite the above mentioned advances, there are still no effective and simple methods for treating DEB. In particular, in those cases where DEB is caused by PTC type of mutation in the COL7A1 gene, it would be ideal to offer patients with a simple therapy that will reverse the PTC phenotype.

Therefore, there still exists a great need for better method of treating skin wounds in general and DEB in particular.

### SUMMARY OF THE INVENTION

In view of the above, it is an object of the present invention to provide a composition for treating DEB caused by premature termination codon mutation in the *COL7A1* gene.

These and other objects of the present invention are satisfied by the unexpected discovery that aminoglycoside could counter the effect of nonsense mutation by inducing the ribosome to read-through the mutation and express the full-length C7 or increase expression of truncated C7. Based on the unexpected discoveries of the invention, various compositions for enhancing wound healing have been devised.

The scope of the invention is defined by the appended claims. Generally disclosed herein is a method for inducing expression of full-length C7 or enhancing expression of truncated C7 in a cell containing nonsense mutation in the COL7A1 gene. Methods in accordance with embodiments of this aspect will generally include the step of contacting the cell with an aminoglycoside.

In another aspect disclosed herein is a method for treating a subject suffering from DEB caused by nonsense mutation in the COL7A1 gene. Methods in accordance with this aspect will generally include the step of administering to the subject a composition comprising an effective amount of aminoglycoside.

In one aspect, the present invention provides a composition for treating DEB as defined in the appended claims. Embodiments in accordance with this aspect of the invention will
generally include an aminoglycoside; C7, mini-C7, or both; and a pharmaceutically acceptable carrier.

The compositions of the present invention will have at least the advantage that they are easy to administer, does not require site-specific application, inexpensive, and effective.

Other aspects and advantages of the invention will be apparent from the following description and the appended claims.

### BRIEF DESCRIPTION OF THE DRAWINGS

**Figure 1** shows that aminoglycosides are capable of inducing expression of full-length C7 in cells containing nonsense mutations in the COL7A1 gene.
**Figure 2** shows that aminoglycoside induced expression of full-length C7 in cells containing nonsense mutations is dose dependent.
**Figure 3** shows that aminoglycoside is capable of inducing sustained expression of full-length C7 in cells containing nonsense mutations.
**Figure 4** shows that aminoglycoside is capable of inducing sustained expression of full-length C7 in RDEB keratinocytes.
**Figure 5** demonstrates that aminoglycoside-induced expression of full-length C7 is also capable of reversing keratinocyte hypermobility.
**Figure 6** shows a schematics view of the nonsense mutations introduced into the *COL7A1* gene of an experimental model using site-directed mutagenesis.
**Figure 7** shows two exemplary aminoglycosides that are capable of inducing read-through of nonsense mutations and production of full-length C7 in a variety of nonsense mutations.
**Figure 8** shows that a large number of cells containing nonsense mutation are capable of expressing truncated C7 and aminoglycosides are capable of enhancing expression level of truncated C7 in these cells.

### DETAILED DESCRIPTION

The present invention will now be described in detail by referring to specific embodiments as illustrated in the accompanying figures. Although the following description is in terms of specific exemplary embodiments and examples, it will be appreciated that the embodiments disclosed herein are for illustrative purposes only and various modifications and alterations might be made by those skilled in the art without departing from the spirit and scope of the invention as set forth in the appended claims.

### Definitions

Unless otherwise indicated, all terms used herein have the meanings given below, and are generally consistent with same meaning that the terms have to those skilled in the art of the present invention. Practitioners are particularly directed to Sambrook et al. (1989) Molecular Cloning: A Laboratory Manual (Second Edition), Cold Spring Harbor Press, Plainview, N.Y. and Ausubel F M et al. (1993) Current Protocols in Molecular Biology, John Wiley & Sons, New York, N.Y., for definitions and terms of the art. It is to be understood that this invention is not limited to the particular methodology, protocols, and reagents described, as these may vary.

As used herein, the acronym "C7" stands for collagen type VII encoded by the gene *COL7A1.*

As used herein, the acronym "C7M" stands for "mini-C7" as described by Chen et al. (J. Biol. Chem. 275(32):24429-24435 (2000), the content of which is incorporated herein by reference.) For the purpose of this invention, "C7M" and "mini-C7" are used interchangeably. Briefly, C7M is formed by selectively removing a portion from the wild-type C7. Wild-type C7 is a protein consisting of 2,944 amino acid residues. It can be further divided into the non-collagenous NC1 domain (residues 1-1253), the central collagenous helical domain (residues 1254 - 2783), and the carboxyl-terminal NC2 domain (residues 2784 - 2944). C7M is formed by removing residues 1920 - 2603 within the central collagenous domain.

As used herein, the term "RDEB" means recessive dystrophic epidermolysis bullosa, which encompasses both Hallopeau-Siemens type RDEB (HS-RDEB) and non-Hallopeau-Siemens type RDEB (non-HS RDEB).

As used herein, the term "truncated C7" refers to a protein expressed from a COL7A1 gene containing a nonsense mutation. There are a great number of potential places where a nonsense mutation can occur in the COL7A1 gene. When this mutant gene is expressed, the resulting protein will be a shortened version that prematurely terminates at the position corresponding to the mutation. Therefore, in this context, truncated C7 is taken as to encompass the collection of all possible shortened C7 due to nonsense mutations.

### Methods for inducing expression of full-length C7 or enhancing expression of truncated C7

Disclosed herein is a method for inducing expression of full-length C7 or enhancing expression of truncated C7 in a cell containing nonsense mutation in the COL7A1 gene. Methods in accordance with embodiments of this aspect will generally include the step of contacting the cell with an aminoglycoside.

Aminoglycosides are amino-modified sugars. Many of them are known to function as antibiotics. Those known in the art may be obtained from commercial sources. Exemplary aminoglycosides may include gentamicin, and paromomycin, but are not limited thereto.

Preferably, the aminoglycoside is selected from the group consisting of G418, gentamicin, and paromomycin. More preferably embodiment, the aminoglycoside is G418.

The cell is preferably a keratinocyte, more preferably a DEB or RDEB keratinocyte.

### Method for treating DEB due to nonsense mutation in the COL7A1 gene

In another aspect, a method for treating a subject suffering from DEB caused by nonsense mutation in the COL7A1 gene is disclosed. Methods in accordance with this aspect will generally include the step of administering to the subject a composition comprising an effective amount of aminoglycoside.

Exemplary aminoglycoside may include, but are not limited to gentamicin, and paromomycin. Preferably, the aminoglycoside is G418.

The composition may further comprise C7 or mini-C7 or both to further assist wound healing.

Most of administration is not particularly limited, but preferably is in a mode that is most common for the particular aminoglycoside. In a preferred embodiment, the composition is one including C7, mini-7, or both, and the mode of administration is topical application.

### Compositions for treating DEB

In one aspect, the present invention provides a composition for treating DEB according to claim 1. Also, the present invention provides a composition for use in enhancing skin wound healing according to claim 4. Embodiments in accordance with this aspect of the invention will generally include an aminoglycoside; C7, mini-C7, or both; and a pharmaceutically acceptable carrier.

In a preferred embodiment, the aminoglycoside is one selected from G418 gentamicin, and paromomycin, more preferably G418.

To further illustrate the present invention, the following specific examples are provided

### EXAMPLES

### Aminoglycosides are capable of inducing expression of full-length C7 in cells containing nonsense mutations in the COL7A1 gene

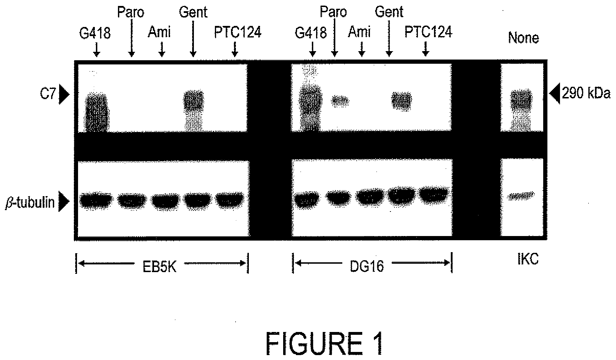

RDEB keratinocytes were treated for 48 h with either G418 (8 µg/mL), gentamicin (200 µg/mL), paromomcyin (200 µg/mL), amikacin (1 mg/mL), or PTC124 (20 µg/mL). Cells were subsequently lysed and subjected to immunoblot analysis with anti-NCI antibody or anti-β-tubulin antibody (loading control). Note in **Figure 1** that treatment with G418 and gentamicin induced full-length C7 production in both EB5K and DG16 keratinocytes. Paromomycin induced read-through in DG16 cells only, while Amikacin and PTC124 did not cause read-through in either keratinocyte line. Note that a single dose of the treatment drug resulted in read-through and produced C7 at a level of 10-35% of that observed in normal human keratinocytes (IKC).

### Aminoglycoside induced expression of full-length C7 in cells containing nonsense mutations is dose dependent

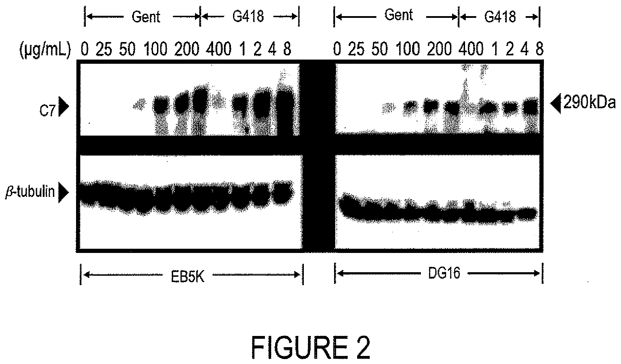

RDEB keratinocytes were treated with varying concentrations of G418 and Gentamicin. Cells were lysed and subjected to immunoblot analysis with an anti-NCI or anti-β-tubulin antibody (loading control). Note in Figure 2 that both G418 and gentamicin induced full-length C7 production in a dose-dependent manner in both EB5K and DG16 lines.

### Aminoglycoside is capable of inducing sustained expression of full-length C7 in cells containing nonsense mutations

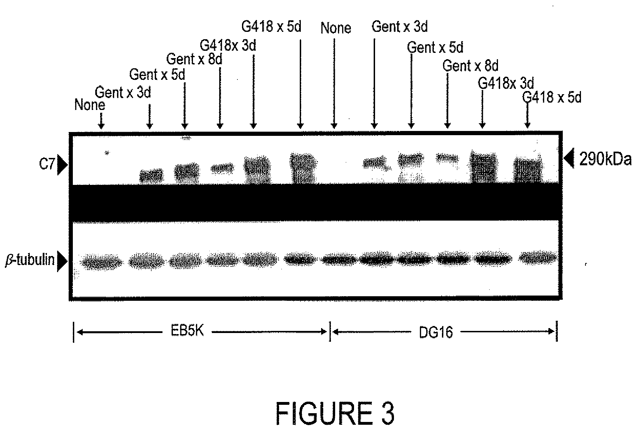

EB5K and DG16 keratinocytes were treated with G418 (8 µg/mL) or gentamicin (200 µg/mL). Cells were lysed at 3, 5, or 8 days following treatment and subjected to immunoblot analysis with an anti-NC1 or anti-β-tubulin antibody. Note in Figure 3 that both gentamicin and G418 induced sustained C7 production.

### Aminoglycoside is capable of inducing sustained expression of full-length C7 in RDEB keratinocytes

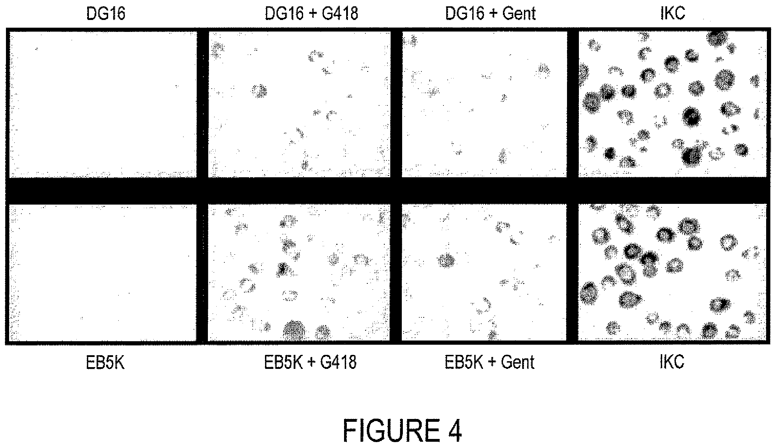

DG16 and EB5K keratinocytes were treated with G418 (8 µg/mL) or gentamicin (200 µg/mL) as indicated in **Figure 4****.** Immunofluorescence staining was performed using a polyclonal anti-NC1 antibody. Normal human keratinocytes (IKC) were stained in parallel. Note that G418 or gentamicin-treated DG16 and EB5K keratinocytes demonstrated C7 staining. In contrast, untreated parent keratinocytes entirely lacked C7 expression.

### Aminoglycoside-induced expression of full-length C7 is also capable of reversing keratinocyte hypermobility

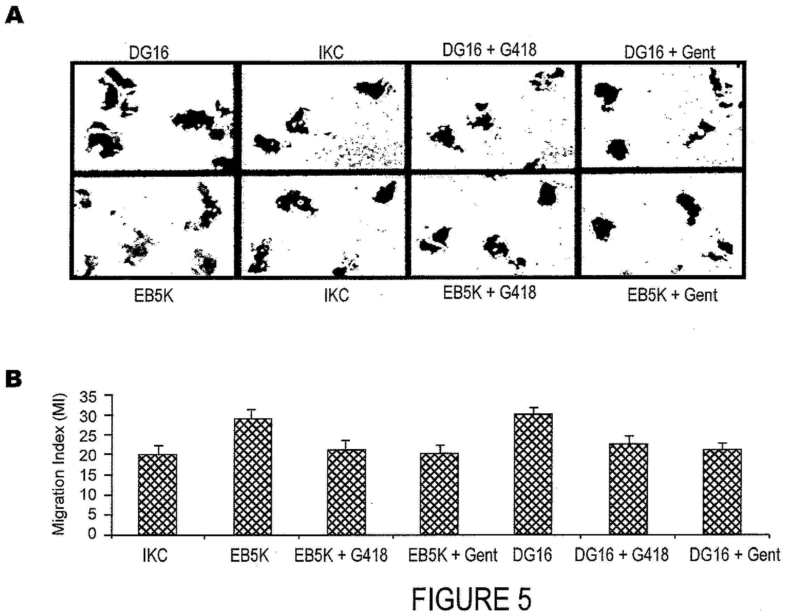

**Figure 5** shows that aminoglycoside-induced expression of full-length C7 is capable of reversing keratinocyte hypermobility, a key feature that is effective in enhancing wound healing. In **Figure 5A****,** keratinocytes were either untreated or treated with G418 (8 µg/mL) or gentamicin (200 µg/mL) for 48 h and then subjected to colloidal gold salt migration assay using collagen I as a matrix. Representative fields were photographed at 40X under dark field optics. In **Figure 5B****,** migration index expresses the percentage of the total field area consumed by the migration tracks. Error bars, SD of three different experiments. Note that EB5K and DG16 keratinocytes showed hypermotility in compared with normal human keratinocytes (IKC). In contrast, aminoglycoside treatment reversed their hypermotility phenotype to normal.

### Construction of COL7A1 nonsense mutants by site-directed mutagenesis

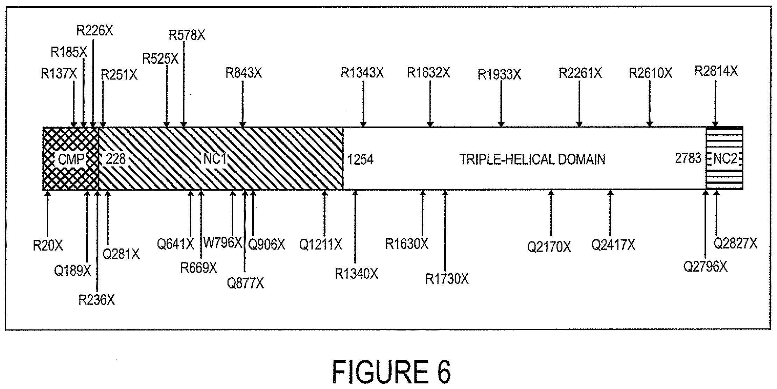

For experimentation with cells containing different nonsense mutation in the *COL7A1* gene, we used a C7 expression vector to introduce 30 nonsense mutations associated with RDEB via site-directed mutagenesis. **Figure 6** is a schematic of the C7 molecule demonstrating the location of each of the newly introduced mutations.

### Aminoglycosides are capable of inducing read-through of nonsense mutations and production of full-length C7 in a variety of nonsense mutations

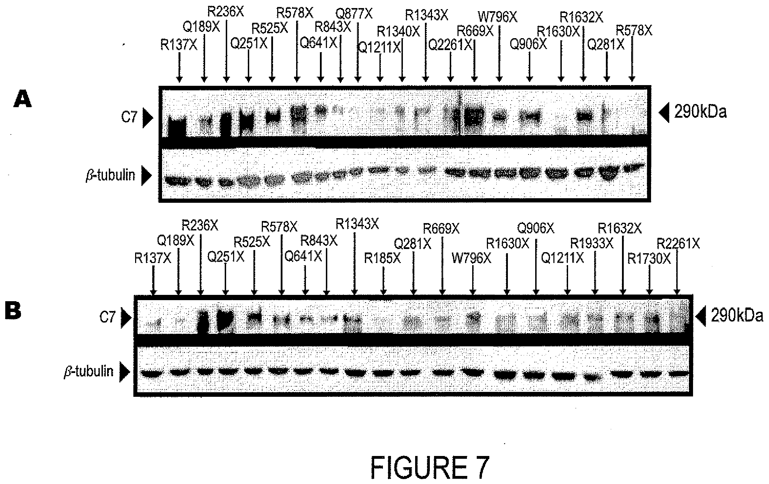

**Figure 7** shows that aminoglycosides are capable of inducing read-through of nonsense mutations, which lead to the expression of full-length C7. Human epithelial (293 cells) transfected with nonsense mutation constructs were treated with (**Figure 7A**) G418 (8 µg/mL) and (**Figure 7B**) gentamicin (200 µg /mL) for 48 h, lysed, and subjected to immunoblot analysis with an anti-NC1 antibody or anti-β-tubulin antibody (loading control). Note that both G418 and gentamicin induced PTC read-through and full-length C7 production in the cell lines containing the indicated mutant construct.

### A large number of cells containing nonsense mutation are capable of expressing truncated C7 and aminoglycosides are capable of enhancing expression level of truncated C7 in these cells

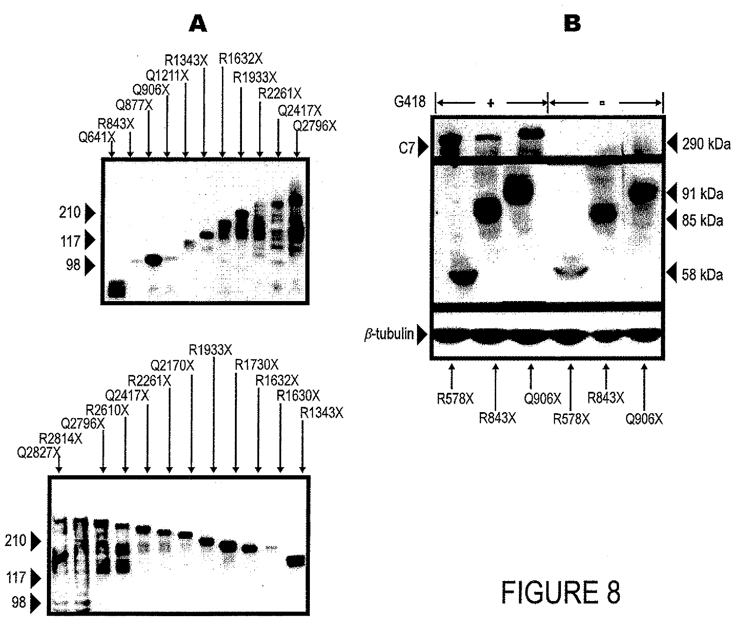

**Figure 8** shows a surprising discovery that all of the constructed nonsense mutants express truncated C7. This is in unexpected because conventional view would predict that nonsense mutation will result in no expression in some cells. In **Figure 8A****,** 293 cells transfected with the indicated mutant constructs were lysed and subject to immunoblot analysis using an anti-NC1 antibody. Note that, in the absence of any treatment, cells harboring PTC mutations produced truncated C7.

In Figure 8B, 293 cells transfected with the indicated mutant constructs were either untreated or treated with G418 (8 µg/mL) for 48 h, lysed and subjected to immunoblot analysis using an anti-NCI antibody or anti- β-tubulin antibody (loading control). Note that cells treated with aminoglycoside demonstrated significantly higher levels of truncated and full-length C7 production when compared to untreated cells.

## Claims

1. A composition comprising (i) an aminoglycoside and (ii) type VII collagen (C7), mini-C7, or both for use in a method for treating a subject suffering from dystrophic epidermolysis bullosa (DEB) due to a nonsense mutation in the COL7A1 gene selected from the group consisting of R20X, R137X, Q189X, R236X, Q281X, R525X, R669X, W796X, and R1632X, the method comprising:
administering to said subject said composition comprising an effective amount of (i) an aminoglycoside selected from the group consisting of G418 and gentamicin and (ii) C7, mini-C7, or both by topical application.

2. The composition for use according to claim 1, wherein said aminoglycoside is G418.

3. A composition for use in enhancing skin wound healing in a subject suffering from DEB due to a nonsense mutation in the COL7A1 gene selected from the group consisting of R20X, R137X, Q189X, R236X, Q281X, R525X, R669X, W796X, and R1632X, the composition comprising:
an aminoglycoside selected from the group consisting of G418 and gentamicin;
C7, mini-C7, or both, and
a pharmaceutically acceptable carrier.

4. The composition for use according to claim 3, wherein said aminoglycoside is G418.

## Patentansprüche

1. Zusammensetzung, umfassend (i) ein Aminoglycosid und (ii) ein Typ-VII-Kollagen (C7), mini-C7, oder beide, zur Verwendung in einem Verfahren zum Behandeln eines Patienten, der an dystrophischer Epidermolysis bullosa (DEB) aufgrund einer Nonsense-Mutation im COL7A1-Gen leidet, ausgewählt aus der Gruppe bestehend aus R20X, R137X, Q189X, R236X, Q281X, R525X, R669X, W796X und R1632X, wobei das Verfahren umfasst:
Verabreichen der Zusammensetzung an den Patienten, die eine wirksame Menge (i) eines Aminoglycosids, das aus der Gruppe bestehend aus G418 und Gentamycin ausgewählt ist und (ii) C7, mini-C7, oder beide umfasst, durch topische Applikation.

2. Zusammensetzung zur Verwendung nach Anspruch 1, wobei das Aminoglycosid G418 ist.

3. Zusammensetzung zur Verwendung bei der Verbesserung der Hautwundheilung in einem Patienten, der an DEB aufgrund einer Nonsense-Mutation im COL7A1-Gen leidet, ausgewählt aus der Gruppe, bestehend aus R20X, R137X, Q189X, R236X, Q281X, R525X, R669X, W796X und R1632X, wobei die Zusammensetzung umfasst:
ein Aminoglycosid, das aus der Gruppe bestehend aus G418 und Gentamycin ausgewählt ist;
C7, mini-C7, oder beide, und
einen pharmazeutisch annehmbaren Träger.

4. Zusammensetzung zur Verwendung nach Anspruch 3, wobei das Aminoglycosid G418 ist.

## Revendications

1. Composition comprenant (i) un aminoglycoside et (ii) du collagène de type VII (C7), mini-C7, ou les deux destinés à être utilisés dans un procédé de traitement d'un sujet souffrant d'épidermolyse bulleuse dystrophique (EBD) causée par une mutation non-sens dans le gène COL7A1 choisi dans le groupe constitué de R20X, de R137X, de Q189X, de R236X, de Q281X, de R525X, de R669X, de W796X et de R1632X, le procédé comprenant :
l'administration audit sujet de ladite composition comprenant une quantité efficace (i) d'un aminoglycoside choisi dans le groupe constitué de G418 et de la gentamicine et (ii) de C7, mini-C7, ou des deux à l'aide d'une application topique.

2. Composition destinée à être utilisée selon la revendication 1, dans laquelle ledit aminoglycoside est le G418.

3. Composition destinée à être utilisée dans l'amélioration de la cicatrisation d'une blessure de la peau chez un sujet souffrant d'EBD causée par une mutation non-sens dans le gène COL7A1 choisi dans le groupe constitué de R20X, de R137X, de Q189X, de R236X, de Q281X, de R525X, de R669X, de W796X et de R1632X, la composition comprenant :
un aminoglycoside choisi dans le groupe constitué de G418 et de la gentamicine ;
du C7, mini-C7, ou les deux, et
un excipient pharmaceutiquement acceptable.

4. Composition destinée à être utilisée selon la revendication 3, dans laquelle ledit aminoglycoside est le G418.
